(19) Europäisches Patentamt
European Patent Office
Office européen des brevets

(11) EP 4 613 185 A1

(12) **EUROPEAN PATENT APPLICATION**

(43) Date of publication:
10.09.2025 Bulletin 2025/37

(21) Application number: 24161670.5

(22) Date of filing: 06.03.2024

(51) International Patent Classification (IPC):
*A61B 5/022* (2006.01)   *A61B 5/024* (2006.01)
*A61B 5/00* (2006.01)   *A61B 5/021* (2006.01)

(52) Cooperative Patent Classification (CPC):
**A61B 5/02108; A61B 5/02125; A61B 5/02416;**
**A61B 5/352; A61B 5/7246; A61B 5/7278;**
A61B 5/022; A61B 5/02225; A61B 5/6824;
A61B 2560/0223; A61B 2560/0238

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventor: **TEN KATE, Warner Rudolph Theophile**
**Eindhoven (NL)**

(74) Representative: **Philips Intellectual Property &**
**Standards**
**High Tech Campus 52**
**5656 AG Eindhoven (NL)**

(54) **DEVICE, SYSTEM AND METHOD FOR CALIBRATING A BLOOD PRESSURE MEASUREMENT FUNCTION**

(57)    The present invention relates to device, system and method for calibrating a blood pressure, BP, measurement function. The device comprises a pressure input (51) configured to obtain time-dependent cuff pressure values during inflation and/or deflation of a cuff (11) of a pressure-delivery system (10) attached to a subject's body part; a sensor input (52) configured to obtain a first time-dependent sensor signal and a second time-dependent sensor signal, both related to the subject's heartbeat and measured during the inflation and/or deflation of the cuff; and a processing unit (53) configured to calibrate the blood pressure measurement function based on a reference BP value, a computed pulse-related value and a computed derivative value of the derivative of the fitting function at the reference BP value.

FIG.8

EP 4 613 185 A1

**Description**

FIELD OF THE INVENTION

The present invention relates to device, system and method for calibrating a blood pressure (BP) measurement function, in particular a BP measurement function that uses a BP surrogate instead of a real BP measurement for use in monitoring or measuring a subject's blood pressure.

BACKGROUND OF THE INVENTION

The traditional method to measure Blood Pressure (BP) at home is the cuff-based system. By inflating the cuff, the cuff pressure (CP) values are determined when they match the diastolic (DBP) and systolic (SBP) pressure. During the inflation the cuff's pressure signal includes a small pulsing, induced by the heart's beating. The pulses are audible by using a stethoscope, known as the Korotkoff sounds. Roughly, the points during the inflation where these Korotkoff sounds appear and disappear correspond with the diastolic blood pressure (DBP) and systolic blood pressure (SBP), known as auscultatory method. Similarly, the growth and decline of the superimposed pulsing in the pressure signal can be used to determine the DBP and SBP, known as oscillatory method.

Wearable solutions to measure BP non-invasively are arising. They bear the promise of easier and more comfortable usage and the possibility to measure continuously. Typically, these solutions use a PPG (Photoplethysmography) sensor, generally comprising a pair of (one or more) LEDs and (one or more) photodiodes, that measures the light reflection by the underlying tissue or transmission through the underlying tissue, including one or more pulsing arteries. The pulsing modulates the intensity of the reflected (or transmitted) light. If reference will be made to the use of reflected light in the following, this does not mean that the use of transmitted light is excluded. Other modalities (including tonometry or ultrasound) to sense the pulsing are known. Both the pulse's morphology and the time the pulse takes to transit the arteries depend on BP, and both can be used to estimate the BP. For obtaining the transit time, a second PPG sensor, most often located elsewhere, or an ECG (Electrocardiogram) sensor is used, such that a (transit) path exists between the two pulses. Other modalities are conceivable, such as sound-based (BCG (ballistocardiogram) and SCG (seismocardiogram)), tonometry and ultrasound.

In general, for the wearable solutions, calibration to the user is required to obtain sufficient accuracy in the BP measurement. This relates to the fact that the pulse transmission depends on other factors (varying with the user) than BP alone, for example the actual elasticity of the artery and its average diameter, the artery wall's thickness, the length of the artery, etc. In fact, BP modulates elasticity and through that the transmission. Further, the elasticity alters with age and requires (re)calibration over time.

There are, however, problems in realizing the calibration. One problem is that the method should be user-friendly and easy to perform, in order to be acceptable for (at home) usage. The other problem is that the calibration procedure needs to observe a range of BP values, in order to determine the optimal calibration parameters in an accurate manner.

An approach for continuous non-invasive blood pressure measurements is based on BP surrogates. BP surrogates are, e.g., well-known parameters such as the pulse transit time (PTT) and pulse arrival time (PAT), pulse wave velocity, pulse shape, pulse landmarks (such as onset, point of steepest ascent, peak), pulse characteristics (such as rise time, amplitude, width, dicrotic notch, etc.), characteristics extracted through pulse decomposition, or combinations thereof. With these parameters blood pressure can be inferred non-invasively and continuously without using external (cuff) pressures except for reference or initialization purposes, often called calibration. Calibration is a key issue for practical applications in clinical and home settings. Thus, the BP surrogate generally represents a measured quantity (not being blood pressure), from which blood pressure can be estimated/predicted/inferred.

WO 2023/072842 A1 discloses a device, system and method for calibrating a blood pressure, BP, surrogate for use in monitoring a subject's blood pressure. The device comprises a BP input, a sensor input and a processing unit that is configured to compute, during inflation of the cuff, pulse-related values from the first and second time -dependent sensor signals using a first feature in the first time-dependent sensor signal and a second feature in the second time-dependent sensor signal, the pulse-related values being pulse arrival time, PAT, values or pulse transit time, PTT, values, selecting pairs of pulse-related values and corresponding cuff pressure values for computing a calibration parameter for a BP surrogate, a pair comprising a pulse-related value and a corresponding, temporally related cuff pressure value, wherein only pairs of pulse-related values and corresponding cuff pressure values are selected for which one or more predetermined conditions with respect to BP and/or cuff pressure are fulfilled, and computing a calibration parameter for a BP surrogate from the BP measurement value and the selected pairs of pulse-related values and corresponding cuff pressure values.

## SUMMARY OF THE INVENTION

It is an object of the present invention to provide a device, system and method enabling a further improvement in the accuracy of continuous non-invasive BP measurements using BP surrogates.

In a first aspect of the present invention a device for calibrating a blood pressure, BP, measurement function is presented, the device comprising:

a pressure input configured to obtain time-dependent cuff pressure values during inflation and/or deflation of a cuff of a pressure-delivery system attached to a subject's body part;

a sensor input configured to obtain a first time-dependent sensor signal and a second time-dependent sensor signal, both related to the subject's heartbeat and measured during the inflation and/or deflation of the cuff; and

a processing unit configured to:

- compute pulse-related values from the first and second time-dependent sensor signals measured during inflation and/or deflation of the cuff, using a first feature in the first time-dependent sensor signal and a second feature in the second time-dependent sensor signal, the pulse-related values being pulse arrival time, PAT, values or pulse transit time, PTT, values,
- determine a fitting function and its derivative between the pulse-related values and the cuff pressure values from selected pairs of pulse-related values and corresponding temporally related cuff pressure values,
- compute the pulse-related value and a derivative value of the derivative of the fitting function at a reference BP value, and
- calibrate the blood pressure measurement function based on the reference BP value, the computed pulse-related value and the computed derivative value of the derivative of the fitting function at the reference BP value.

In a further aspect of the present invention a system for calibrating a blood pressure, BP, measurement function is presented, the system comprising:

a pressure-delivery system comprising a cuff configured to be attached to the subject's body part and to deliver a pressure to the subject's body part by inflating the cuff;

a pressure sensor configured to acquire time-dependent cuff pressure values during inflation and/or deflation of a cuff of the pressure-delivery system attached to the subject's body part and to acquire or infer a reference BP value;

a first sensor configured be attached to a first site of the subject's body and to acquire a first time-dependent sensor signal related to the subject's heartbeat during the inflation and/or deflation of the cuff;

a second sensor configured be attached to a second site of the subject's body and to acquire a second time-dependent sensor signal related to the subject's heartbeat during the inflation and/or deflation of the cuff; and

a device as disclosed herein for calibrating the blood pressure measurement function from the reference BP value, the time-dependent cuff pressure values and the first and second time-dependent sensor signals.

In yet further aspects of the present invention, there are provided a corresponding method, a computer program which comprises program code means for causing a computer or processor to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention enables reliable personalized calibration procedures (sometimes also called initiation or initialization procedures) utilizing cuff inflations and/or deflations to induce well-defined associated changes in blood pressure surrogates. Insights on physiological effects during cuff inflation and/or deflation resulted in optimized methods and procedures used for computing the one or more calibration parameters. The reliably calibrated blood pressure surrogates enable accurate non-invasive estimation tracking/monitoring of blood pressure, i.e., by use of the calibrated BP surrogate an - initially open / undefined - set of parameters can be translated into the meaningful clinical parameter blood pressure.

The present invention is based on the finding that changes in PAT (or PTT) (or any other BP surrogate(s)) during cuff pressurization are distorted by dynamic filling effects in the lower arm (or, more generally, in a peripheral region of the subject's body part at which the cuff is arranged) due to effects like venous occlusion, changes of the elasticity of the artery beneath the cuff, and/or (partial) occlusion of the artery itself. In first order, these effects may be taken together as changing PAT/PTT in dependence on CP. This finding affects the way how to obtain one or more calibration parameters. For instance, parameter regression - as one preferred option to compute the one or more calibration parameters - is affected, which may be addressed for the inference of a sensitivity parameter. The effect has been observed and interpreted from invasively measured BP signals acquired peripheral to the cuff, e.g., in the lower arm when the cuff is arranged at the upper arm, or in the lower leg when the cuff is arranged at the upper leg.

In fact, the complexity of this conglomerate of reasons for changes of PAT or PTT is one reason for observing the (fitted) curve at the reference BP value. Furthermore, the dependence between BP and PAT/PTT is of interest, while the data reflect the dependence between PAT/PTT and CP. At that reference BP point the effects start taking off, the complexity of their interactions is not yet happening, and by that (the derivative(s) of) the fitting curve (between PAT/PTT and CP) matches the derivative(s) of the dependence between BP and PAT/PTT that shall be calibrated.

Thus, according to the present invention, BP metrics are measured during inflation and/or deflation. It is known that these metrics vary with the CP in the inflating cuff. While these BP metrics vary, it is not the BP values themselves that vary. By extracting the trend in these varying BP metrics (in dependence on CP), and use that to estimate their dependence with BP, i.e., how they predict the user's BP, a more accurate calibration can be realized. This dependence between PAT/PTT and CP is the reverse (and reciprocal) of the to be determined trend between BP and PAT/PTT. In other words, calibrating the predicting of BP from these metrics (e.g., PAT/PTT) comes from extracting and reverting this metrics dependence on CP. In this way, the BP measurement can be calibrated in a user-friendly manner while still accounting for a varying BP range. Instead of calibrating at a single point (since no varying BP is available), at least two data points are made available by the approach of the present invention, and the accuracy of the calibration can be improved. By including higher order derivatives of the fitting function more data points may be made available and be used according to the present invention.

The present invention may be applied in ambulatory scenarios or as part of a telemonitoring setting. For instance, in home monitoring, telemonitoring or holter monitoring settings, there is an interest to provide convenience (e.g., replacing regular self-measurement with a cuff; replacing the ambulatory blood pressure monitoring (ABPM) which may be quite disturbing or annoying to patients, to have continuous measurement (preferably adjusted for activity and posture), to measure short-time variability, to measure nocturnal dips (BP drops during sleep in healthy people), and to supplement holter monitors (ECG sensing for cardiac diagnosis). The device, system and method of the present invention enable to achieve one or more of these goals.

The present invention allows to calibrate a BP measurement function, which may, e.g., be a BP prediction model or BP estimation algorithm, so that based on BP surrogate, i.e., some metric/measure, BP estimates can be predicted more reliably and accurately. In practice, this calibration may appear as if a particular device (measuring the surrogate values) or method is calibrated, i.e., the measurement procedure hosted by that device, or hosted in the cloud using data from that device. Calibration may also relate to the particular user wearing the device.

In first order, pressure over the arterial wall, the so-called transmural pressure, equals the difference BP-CP. The pulse propagation behaves differently with different transmural pressure, i.e., with different CP (assuming BP to be stationary over the measurement duration). Pressure differences may drop over other parts of the tissue, but this is neglected (or assumed a constant).

The present invention preferably measures during inflation because the vascular system responds in a more stable way, in another embodiment measurement during deflation or during both phases may be used. In yet another embodiment, the cuff is inflated (and deflated) several times, so that more data points are measured. A rest period of at least a couple of seconds is preferably present between deflation and next inflation. The inflation and deflation can be at constant rate but may also vary. Stepwise inflation/deflation is also envisioned in an embodiment.

The second feature in the second time-dependent sensor signal may be of the same nature or type as the first feature in the first time-dependent sensor signal (e.g., when both signals are PPG signals, or when both stem from two sub-pulses derived by pulse decomposition).

The sites for measuring the first time-dependent sensor signal and the second time-dependent sensor signal may be identical or different. If one of the signals is an ECG signal, that signal may be measured at the same location as that of the PPG (supposed to be the other signal). Since ECG pulses travel much faster, the characteristic time point of every beat arrives earlier, as if the ECG sensor was located near the heart. If the two time-dependent signals result from pulse decomposition of a measured, e.g., PPG-signal, the measuring sites can be a single one. Using multiple sites and fusing the results from them may raise accuracy. Thus, measuring at two locations (like two PPG sensors or a PPG and separate (continuous) ECG sensor) and measuring at one location (like PPG and ECG within watch and single PPG where the two sensor signals are extracted by (two) different processing on the PPG signal/pulse, e.g., extracting a first sub-pulse and second sub-pulse stemming from pulse decomposition of the measured PPG pulse are two options of implementing the present invention covered by the claims.

In an embodiment, the processing unit is configured to obtain or determine the diastolic BP, DBP, as reference BP value, in particular to determine DBP from selected pairs of pulse-related values and corresponding temporally related cuff pressure values or to obtain DBP from an internal measurement by the cuff. This allows a simple computation of a reference BP value that can be used in the calibration.

In another embodiment the processing unit is configured to calibrate a chosen BP prediction mode having an intercept and a slope, wherein an intercept of the BP prediction model is determined by a pulse-related value at the reference BP value and a slope of the BP prediction model at said intercept is determined by from the slope of the fitting curve at the reference BP value, in particular by the reverse inverted (reciprocal) and negated slope of the fitting curve at the reference BP value.

According to an embodiment the processing unit is configured to use only pairs of pulse-related values and corresponding cuff pressure values for computing the calibration parameter for the BP measurement function that have been computed from the first and second time-dependent sensor signals measured during a partial inflation of the cuff. It has been found that at high cuff pressure no reliable pulse signals due to pulse distortions appear in peripheral part of the body part at which the cuff is arranged, which may negatively affect the computation of the calibration parameter if pairs of values are used that are computed from sensor signals measured during a fully or almost fully inflated cuff. These negative effects are avoided by this embodiment since only pulse signals with defined relationships to BP are taken into account, which will improve accuracy of the calibration and thus finally improve BP determination by use of the calibrated BP measurement function.

According to another embodiment the processing unit is configured to use only pairs of pulse-related values and corresponding cuff pressure values for computing the calibration parameter for the BP measurement function for which a peripheral BP is substantially constant, in particular varies by less than 10 % or less than 5 %. Other reasonable values may be used as well. Similar effects can be achieved in this way as explained above, although a different condition is used for selecting pairs of values for the computation.

The processing unit may hereby be configured to determine if the peripheral BP is substantially constant based on the first and/or second time-dependent sensor signal. For instance, the period during which an amplitude of the second time-dependent signal is substantially constant, in particular varies by less than 10 % or less than 5 % (or any other reasonable threshold that is predetermined or set by user) with respect to a previously acquired average value, may be determined for this purpose. This previously acquired average value refers to a previous average value of the second time-dependent signal.

The processing unit may further be configured to use only pairs of pulse-related values and corresponding cuff pressure values for computing the calibration parameter for the BP measurement function for which the cuff pressure value is below the subject's diastolic BP, in particular the subject's latest measured diastolic BP, or below the subject's mean BP, in particular the subject's latest measured mean BP, or below a set threshold cuff pressure. This enables a rather simple but efficient implementation of the disclosed solution.

According to a further embodiment, the DBP (diastolic BP) and MAP (Mean Arterial Pressure) may be determined from the cuff pressure: either given by the cuff measurement system or by extraction from the pulsing that is superimposed in the CP signal.

In another embodiment the processing unit is configured to use only pairs of pulse-related values and corresponding cuff pressure values for computing the calibration parameter for the BP measurement function for which the cuff pressure value is in a range between a set minimum cuff pressure and a set maximum cuff pressure, in particular wherein the minimum cuff pressure is set in the range of 0 to 40 mmHg and the maximum cuff pressure is set in the range of 40 to 110 mmHg (or in a range defined by other reasonable values). This enables a rather simple but efficient implementation of the disclosed solution as well.

In a still further embodiment, the processing unit may be configured to use only pairs of pulse-related values and corresponding cuff pressure values for computing the calibration parameter for the BP measurement function for which the pulse-related values are substantially constant, in particular vary by less than 10 % (or any other reasonable value), or until the slope of a curve of the pulse-related values over time exceeds a threshold. These embodiments make use of other conditions that can be easily implemented.

The processing unit may further be configured to compute a control signal for controlling the pressure-delivery system to inflate its cuff, in particular to fully inflate the cuff for acquisition of the BP measurement value and to only partly inflate the cuff for acquisition of the first time-dependent sensor signal and the second time-dependent sensor signal. This enables the device to actively control the inflations of the cuff as needed for either a BP measurement or for the acquisition of sensor signals used for determining the calibration parameter. The BP measurement value used for the calibration (i.e. for computing the calibration parameter) may be acquired through BP measurement from a BP measurement device. Next to controlling the range, the pattern of inflation pressures can be controlled, e.g. to implement a constant increment, slow increment, staged, e.g. steps of 5-10 mmHg, where after each step the pressure is kept constant for a couple of seconds, to measure a couple of pulses. The range is preferably restricted to about MAP, such that the total measurement and burden is not unnecessarily expanding.

In preferred embodiments the first time-dependent sensor signal is an ECG signal and/or the second time-dependent sensor signal is a photoplethysmography, PPG, signal, in particular a contact PPG signal or a remotely acquired PPG signal (e.g. acquired by a camera as used in remote PPG), or a multi-color (multi-wavelength) signal (typically green light is used, but red and infra-red might be included to refine the PPG signal). Other signals that are related to the subject's heart rate and allow the computation of PAT and/or PTT can be used in addition or as alternative. For instance, bio-impedance or cuff signals may be used instead of PPG signals. ECG and PPG signals are commonly known signals from which PAT and/or PTT can be computed in a generally known manner that can be used by the device and method of the present invention as well. Further signals that can be acquired non-invasively include ultra-sound, sound (microphone) for BCG and/or SMG, tonometry and accelerometer, gyroscope, magnetometer.

It is further preferred in an embodiment that the processing unit is configured to use as reference BP value one of a value of zero, diastolic blood pressure, systolic blood pressure or mean arterial pressure, and/or to use at least the first-order derivative as derivative of the fitting function (preferably evaluated at that reference BP).

The claimed system may further comprise a control unit configured to control the pressure-delivery system, e.g. based on a control signal provided by the device or computed by the system. The control unit may be part of the device or may be an external entity.

The present invention may also be used in combination with one or more of the following embodiments.

In an embodiment the processing unit is configured to:

- compute a control signal for controlling the pressure-delivery system to repeatedly inflate its cuff in partial inflations up to a cuff pressure below the subject's systolic BP,
- compute pulse-related values from the first and second time-dependent sensor signals, measured during the repeated partial inflations of the cuff, using a first feature in the first time-dependent sensor signal and a second feature in the second time-dependent sensor signal, the pulse-related values being pulse arrival time, PAT, values or pulse transit time, PTT, values, and
- compute a calibration parameter for a BP surrogate from the BP measurement value and pairs of the pulse-related values and corresponding cuff pressure values, computed from the first and second time-dependent sensor signals measured during the repeated partial inflations of the cuff, a pair comprising a pulse-related value and a corresponding, temporally related cuff pressure value.

According to this embodiment only low cuff pressure levels are used for calibration, i.e. the cuff is only partly inflated for data acquisition. Repeated sampling of those relevant cuff pressure levels is feasible in a short enough time period, such that the BP level is stable and does not change, which would distort the calibration process. Furthermore, repeated inflation ramps (with or even without a pause in-between) are enabled by low maximum pressure: there is no need to wait for tissue and arteries to recover from high pressurization (i.e., there is no hysteresis effects).

Low cuff pressure levels, especially cuff pressure levels below diastolic pressure, are very unobtrusive for the patient, particularly for awake patients, resulting in less discomfort and less stress to skin than full inflation ramps up to supra-systolic levels. Another advantage is that it does not pose extra requirements on the pressure-delivery system of a NIBP device.

The BP measurement value used for the calibration (i.e. for computing the calibration parameter) may be acquired through BP measurement from a BP measurement device.

There are different embodiments how to control the pressure-delivery system in the repeated partial inflations. They all try to further avoid the negative dynamic filling effects mentioned above and thus contribute to improving accuracy of the calibration and thus finally improve BP determination by use of the calibrated BP surrogate.

In an embodiment the processing unit is configured to compute the control signal for controlling the pressure-delivery system to inflate its cuff in the partial inflations as long as a peripheral BP is substantially constant, in particular varies by less than 10 percent or less than 5 percent. Other reasonable values may be used as well.

The processing unit may hereby be configured to determine if the peripheral BP is substantially constant based on the first and/or second time-dependent sensor signal, in particular by determining the period during which an amplitude of the second time-dependent signal is substantially constant, in particular varies by less than 10 percent or less than 5 percent (or any other reasonable threshold that is predetermined or set by user) with respect to a previously acquired average value. This previously acquired average value refers to a previous average value of the second time-dependent signal.

According to another embodiment the processing unit is configured to compute the control signal for controlling the pressure-delivery system to inflate its cuff in the partial inflations up to the subject's diastolic BP, in particular the subject's latest measured diastolic BP, up to the subject's mean BP, in particular the subject's latest measured mean BP, or up to a set threshold BP. This enables a rather simple but efficient implementation of the claimed disclosed solution.

The processing unit may be configured to compute the control signal for controlling the pressure-delivery system to inflate its cuff in the partial inflations up to a cuff pressure at which the PAT exceeds a PAT threshold, in particular an absolute PAT threshold or a relative PAT threshold compared to a baseline PAT. Hereby, the processing unit may be configured to compute the baseline PAT by averaging PAT measurements over a period, in particular a period in the range of 10 s to 5 min (other reasonable values may be possible as well). A reasonable threshold for a relative change, i.e. PAT compared to baseline PAT, may for instance be in the range of 10 to 60 ms, e.g. 30 ms.

In another embodiment the processing unit is configured to compute the control signal for controlling the pressure-delivery system to inflate its cuff in the partial inflations up to a cuff pressure at which an amplitude of the second time-dependent signal exceeds an amplitude threshold, in particular an absolute amplitude threshold or a relative amplitude threshold. Such criteria can be easily set by a user or predetermined in advance. A reasonable threshold for a relative change may for instance be in the range of 5 to 20%, e.g. 10%.

The processing unit is configured to compute the control signal for controlling the pressure-delivery system to inflate its cuff in the partial inflations at a predetermined inflation speed or an inflation speed dependent on one or more of the subject's heart rate, the subject's diastolic BP, the subject's mean BP, and the subject's systolic BP.

In another embodiment the processing unit is configured to compute the control signal for controlling the pressure-delivery system to inflate its cuff in a full inflation beyond the subject's systolic BP before and/or after one or more iterations of partial inflation to obtain a time-dependent BP reference measurement for use in monitoring the subject's BP using the BP surrogate. The BP value measured last may particularly be used in the computation of the calibration parameter.

The processing unit may further be configured to control how many iterations of partial inflation are conducted based on a comparison of a regression error to a regression error threshold. Further, it may be configured to control if and when a full inflation is conducted according to a fixed or variable schedule or if one or more calibration parameters substantially changed in the last computation, in particular changed by more than 10 percent (or more than 15 or 20 percent). Thus, a tradeoff may be made between additional measurement time / effort and increased accuracy of the calibration.

In preferred embodiments the first time-dependent sensor signal is an ECG signal and/or the second time-dependent sensor signal is a photoplethysmography, PPG, signal, in particular a contact PPG signal or a remote PPG signal. Other signals that are related to the subject's heart rate and allow the computation of PAT and/or PTT can be used in addition or as alternative. ECG and PPG signals are commonly known signals from which PAT and/or PTT can be computed in a generally known manner that can be used by the disclosed device and method as well.

BRIEF DESCRIPTION OF THE DRAWINGS

These and other aspects of the invention will be apparent from and elucidated with reference to the embodiment(s) described hereinafter. In the following drawings:

Fig. 1 shows a schematic diagram of an embodiment of the general concept of the present invention.
Fig. 2 shows a diagram of an ECG signal and a PPG signal in which PAT is indicated.
Fig. 3 shows diagrams illustrating robust versus non-robust regression in case of a linear model.
Fig. 4 shows a diagram of an example of induced PAT increase during a cuff-inflation.
Fig. 5 shows diagrams of signals of interest in a system according to the present invention.
Fig. 6 shows a diagram of an embodiment of a system according to the present invention.
Fig. 7 shows a diagram of an embodiment of a device according to the present invention.
Fig. 8 shows a diagram of an embodiment of a method according to the present invention.
Fig. 9 shows a diagram of another embodiment of a method according to the present invention.
Fig. 10 shows diagrams of different signals over time used according to an embodiment of the present invention.
Fig. 11 shows a diagram of the pulsing of the cuff pressure.
Fig. 12 shows a typical plot of PAT against CP.

DETAILED DESCRIPTION OF THE INVENTION

The present invention is related to the development of a non-invasive BP (NIBP) measurement technology to enable accurate continuous blood pressure monitoring. A calibration is done using the NIBP measurements themselves (Systolic (SBP), Diastolic (DBP) and mean (MBP) blood pressure) as well as a cuff-driven inflation/deflation process, imposing a cuff-pressure (CP) to the artery system beneath the cuff, which induces controlled CP-related disturbances of the surrogate signals. The surrogates are obtained from features in signals related to the subject's heartbeat such as (preferably) the electrocardiogram (ECG) or photoplethysmogram (PPG) or via their combination or even via other modalities like ultrasound or tonography. These signals are acquired in these setting routinely.

The present invention focuses on the pulse wave velocity methodology towards unobtrusive BP measurement, since sensor embodiments are small and lightweight and offer comfortable quasi-continuous and non-invasive BP measurements. As explained above, wearable solutions to measure BP non-invasively often use a first PPG sensor and, in addition, a second PPG sensor, located elsewhere, or an ECG sensor is used, such that a (transit) path exists between the two pulses. Alternatively, by pulse decompensation techniques two or more sub-pulses are derived from (one or more) PPG signal(s) measured at a single location. The sub-pulses are treated as the pulses above from different sensors (e.g.,

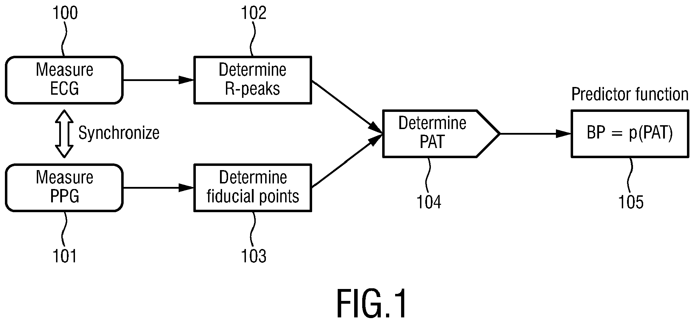

PPG and ECG). Fig. 1 shows a schematic diagram of an embodiment of a measurement scheme according to the present invention, using the PAT (Pulse Arrival Time) method, which is the transit time between, e.g., R-peak in the ECG signal and a fiducial point in the PPG pulse (e.g., foot, tangent intersect, point of steepest ascent, or peak). In this embodiment an ECG signal (first time-dependent sensor signal) and a PPG signal (second time-dependent sensor signal) are measured in a synchronized manner (blocks 100 and 101). From the ECG signal the R-peaks (first feature) are determined (block 102) and from the PPG signal (one or more) fiducial points (second feature) are determined (block 103). Based on the R-peaks and the fiducial points PAT is determined (block 104). The PAT is taken by a predictor function p(PAT) to determine the BP (block 105).

This concept may not need any additional sensors, using available sensors instead, can be implemented in software only, and has a low barrier for clinical acceptance.

An embodiment of the presented approach refers to the use of PAT as BP surrogate. PAT is defined as time interval between the peak of the R wave of the ECG and the onset of a peripheral plethysmographic pulse. It has been intensively investigated as surrogate measure of blood pressure and vessel stiffness. PAT is the sum of the pre-ejection period (PEP) and PTT. PEP refers to the time needed for the iso-volumetric ventricle contraction up to the aortic valve opening (AVO) while PTT is the true transit time of the pressure pulse along the arterial wall over a long non-homogeneous vascular path. The pulse propagation depends on, among others, the (elasticity) properties of the arterial wall, which in turn depends on the transmural pressure. Hence, the PTT depends on arterial pressure. PEP is a varying additive delay sensitive to stress, emotion and physical effort. Major advantage of PAT vs. PTT is that only one location sensitive transducer (e.g. a PPG sensor) needs to be placed accurately.

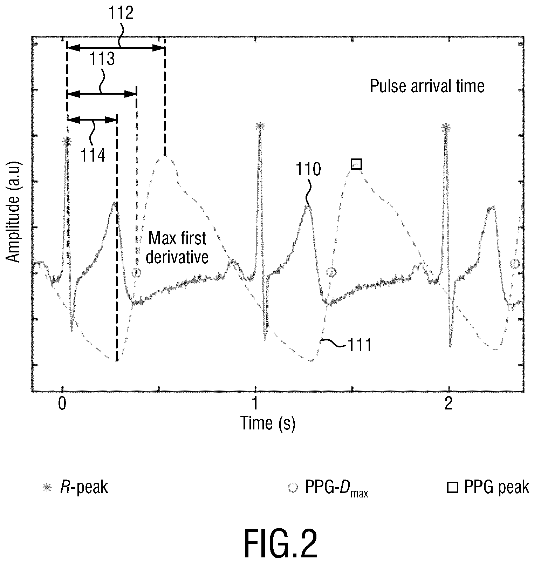

Fig. 2 shows a diagram (partly taken from Shao, J., et al., An Optimization Study of Estimating Blood Pressure Models Based on Pulse Arrival Time for Continuous Monitoring) of an ECG signal 110 and a PPG signal 111 in which PAT is indicated as the time delay between the R-peak in the ECG signal and a characteristic point of a peripheral PPG signal. In this example the maximum derivative ($D_{max}$) is selected as characteristic point. Examples of other characteristic points (also called "fiducial points" or "features") are the foot of the pulse, the tangent intersect, and the peak of the pulse. In general, the peak location exhibits more variance and therefore the peak is less preferred. In Fig. 2, 112 indicates the $PAT_{peak}$, 113 indicates the $PAT_{derivative}$, and 114 indicates the $PAT_{foot}$.

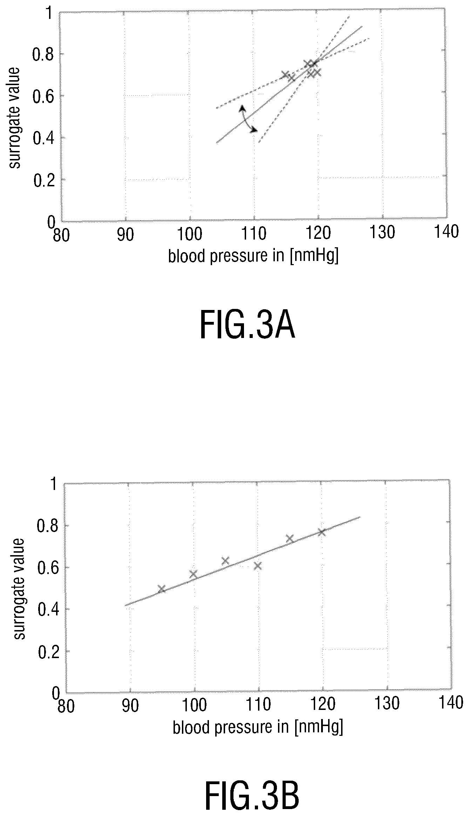

Fig. 3 illustrates how accuracy improves when the fitting is over a wider range of data values. Regression is the typical method to fit a model to data points. In Figs. 3A and 3B two examples are shown where the model is assumed to be a straight line. In case of the blood pressure calibration a curved relationship can be assumed.

A system using PAT to continuously estimate BP does not need any additional devices (hardware), assuming that time-synchronized PPG and ECG sensors (and/or corresponding signals) are available, and therefore, represents a software-only approach to continuous BP estimation.

PAT changes are inversely related to BP changes: if BP increases, the transmural pressure in the arteries increases, leading to a decrease of the arterial wall compliance, i.e., an increase in artery's stiffness (elasticity modulus), which results in faster propagation time of the blood pulse wave and therefore decreased time until arrival of the pulse wave at the periphery. Vice versa, if BP decreases, PAT increases. The exact transfer function from BP to PAT depends on many factors, next to the elasticity, such as the patient's individual arterial properties and geometries, the heart's pre-ejection period (PEP), and others. Furthermore, the transfer function is not static, but varies with time, when the patient's hemodynamic status, i.e. the arterial properties (e.g. arterial smooth muscle or PEP), changes.

However, the transfer function of BP and PAT can be modelled by rather simple models (e.g. proportional linear, logarithmic, inverse, inverse square, and others) with e.g. two or three parameters. For example, considering a simple linear model ($BP = m_1 \cdot PAT + m_2$), the two patient-specific parameters are an offset parameter $m_2$ and a sensitivity parameter (slope) $m_1$. In order to apply the transfer function in practice to continuously estimate BP from continuous PAT values, the patient-specific parameters need to be 'learned'. This is called calibration. Furthermore, changes in these parameters are preferably tracked, i.e., calibration is preferably repeated when the model parameters (i.e. the calibration parameters) are changing. This is called re-calibration.

Typically, calibration of a model is done via parameter regression. I.e., if multiple (BP, PAT) data pairs are available, the model curve can be fitted to the data points by means of applying some optimality criterion (e.g. least squared error), yielding optimal values (for that criterion) for the model parameters based on the provided data. The larger the range of BP and PAT values is, the better (more accurate) are the determined parameters. However, multiple (BP, PAT) data pairs in which the BP values span a large range, may be difficult to realize in a convenient manner, which problem is addressed by the present invention.

In practice, achieving robust calibration of the model is a key challenge to be able to reliably use a BP surrogate for unobtrusive determination and monitoring of a subject's BP. Conducting a regression with multiple BP and PAT measurements obtained in a short time period suffers from the issue that the resulting curve fit will be inaccurate, since the measured BP (and thereby PAT) values do not span a large range. To improve the curve fit, the blood pressure level of the patient would need to be changed, which is typically infeasible without altering stiffness of the arterial wall, may cause harm to the patient, can be unethical, and is unpractical.

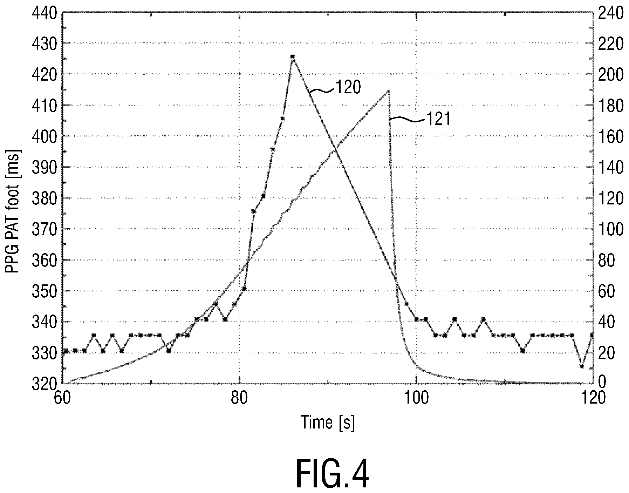

Because of the reduced transmural pressure for the artery segment under the cuff, an increase in PAT can be measured in relation to the applied cuff pressure. This is illustrated in Fig. 4 showing a diagram of an example of induced PAT increase 120 during a cuff-inflation CP 121. The cuff pressure modulates the artery transmural pressure (BP is assumed constant during the inflation). PAT values are obtained for every beat in synchrony with the cuff pressure. More precisely, this sequence applies to the artery segment beneath the cuff. With increasing CP the transmural pressure decreases, which conducts to increased elasticity. This in turn slows down the propagation, and hence extends the segment's transition time. The line from the top of 120 down again does not include measurements. Actual values should be consistent with CP. When inferring BP, a scaling factor may need to be incorporated to account for the full artery length, relative to the cuff-covered segment. It shall be noted that in this system BP does not change, but it is the transmural pressure that changes (by change in CP). The present invention builds on this by assuming continuity at the cross-over (where either BP in/decreases or CP de/increases): the PAT-CP measurement is used to determine the derivative at that cross-over, which is used to calibrate the BP-PAT relationship.

According to the present invention the derivative at the cross-over point (also called "reference BP value", $BP_{ref}$) is determined. Preferably, the derivative is determined from the curve obtained through curve fitting of the measured PAT, CP pairs. The fitting may be done over a restricted regime (for example, up to CP reaching mean arterial pressure (MAP), or up to the cuff pressure where the artery beneath the cuff gets (too much) occluded and/or deformed). The veins get occluded at about 30 mmHg cuff pressure. That means that the blood flow stops (and hence some pressure builds up), but the pulse waves still propagate along the path. Propagation speed is changed with the increased pressure, but also by the omission of the (otherwise superimposed) flow component. Therefore, since in operation there is obviously no occlusion, the fitting range can be lower bounded to exclude this vein-occlusion effect.

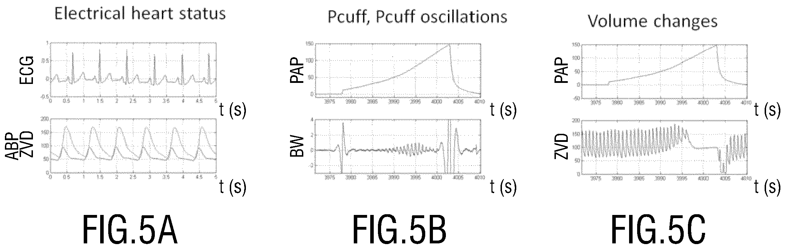

Fig. 5 shows diagrams of signals that may be used in a system according to the present invention. Fig. 5A shows an ECG signal. Fig. 5B shows a cuff pressure signal. Fig. 5C shows a PPG signal.

The current calibration approach (applying cuff pressure to induce PAT changes under the cuff) is affected by non-linear dynamic filling effects in the peripheral arterial tree of the arm. Observed processes can be subdivided into various phases with impact on the mathematical modelling functions and consequences for included/excluded data pairs.

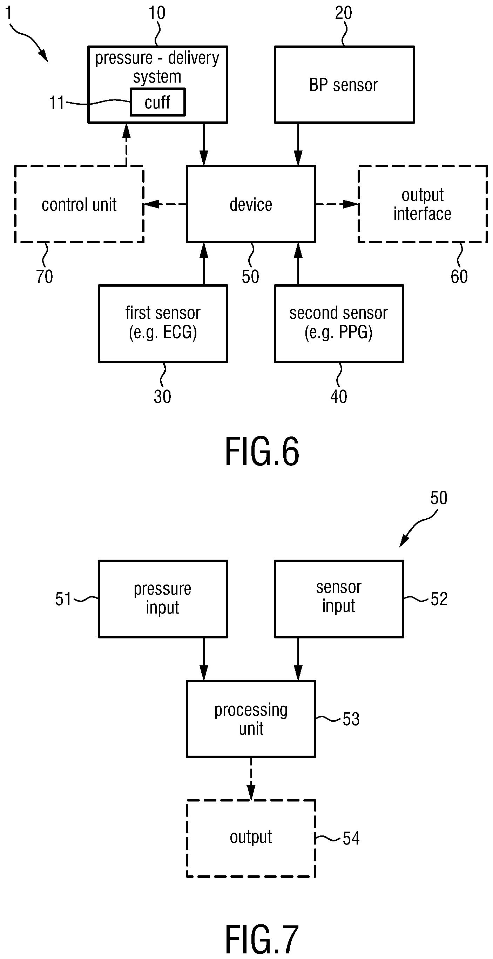

Fig. 6 shows a diagram of an embodiment of a system 1 for calibrating a BP measurement function that uses a BP surrogate for determining / monitoring a subject's blood pressure according to the present invention. The system 1 comprises a pressure-delivery system 10 comprising a cuff 11 configured to be attached to the subject's body part and to deliver a pressure to the subject's body part by inflating the cuff. Such a pressure-delivery system 10 comprising a cuff 11 that can be attached to an extremity of the subject, in particular the upper arm, upper leg or wrist, is generally known in the art of NIBP measurement. Generally, it comprises a pressure generating unit, e.g. a pump or pressure reservoir, that is configured to inflate the cuff 11, a valve that is configured to deflate the cuff 11, and a processor that is configured to control the pressure generating unit and the valve and to determine the subject's blood pressure based on the measured cuff pressure. A user interface may be provided, e.g. comprising one or more of a display, keypad, loudspeaker and touchpad, to issue the measured BP values, e.g. in visible and/or audible form. The sub-system 10 may further be instrumented with an (digital) interface to communicate with the further components in the calibration system. The interface may also serve to synchronize the pressure sensing with the other signals (ECG, PPG, etc.).

The system 1 further comprises a pressure sensor 20 configured to acquire time-dependent cuff pressure values during inflation and/or deflation of the cuff 11 of the pressure-delivery system attached to the subject's body part and to acquire or infer a reference BP value. The DBP, MAP, and SBP, which may be used as reference BP value, can be built-in outcomes from the cuff measurement system, as cuff measurement systems are being used today. The reference BP value generally is a point where the distortions due to the cuff inflation are not yet present. If the fitting of the c(.) function (explained below) is over the data between DBP and MAP (i.e., where CP equals those values), which is the preferred way, the DBP can be taken as the reference BP value. Other examples for the reference BP value may be CP=0 mmHg, or MAP (not preferred), for example.

Typically, the pressure sensor 20 is not directly attached to the subject's body part, but the cuff 11 is attached to the body part and the pressure sensor 20 is attached (at the pressure delivery system) to an air tube that comes from the cuff 11. The pressure sensor 20, e.g. in the form of a pressure transducer, may thus be integrated into the cuff and may be implemented by a conventional BP sensor. Other means to apply an external pressure and to detect cuff pressure, such as a Shellcuff design, are possible as well.

The system 1 further comprises a first sensor 30 configured be attached to a first site of the subject's body and to acquire a first time-dependent sensor signal related to the subject's heartbeat during the inflation and/or deflation of the cuff. The first sensor 30 may be an ECG sensor for acquiring an ECG signal. The first site may e.g. be the subject's chest or torso at which ECG electrodes are preferably attached for ECG measurements.

The system 1 further comprises a second sensor 40 configured be attached to a second site of the subject's body and to acquire a second time-dependent sensor signal related to the subject's heartbeat acquiring signals modulated because of the inflation and/or deflation of the cuff. The second sensor 40 may be a PPG sensor, e.g. a contact PPG sensor (such as a pulse oximetry sensor comprising one or more LEDs emitting light in the visible and/or infrared range, e.g. red and infrared light) or a remote PPG sensor (such as a camera or photo detector, as e.g. known from Verkruysse et al., "Remote plethysmographic imaging using ambient light", Optics Express, 16(26), 22 December 2008, pp. 21434-21445). PPG generally refers to the optical measurement of volume changes of an organ or a body part and in particular to the detection of volume changes due to a cardio-vascular pulse wave traveling through the body of a subject with every heartbeat. Radiation reflected by or transmitted through a skin region of the subject can be detected. The second site may e.g. be the subject's hand or finger at which a contact PPG sensor may be attached, or which may be monitored by a remote PPG sensor. The second site is generally a site peripheral to the first site. For instance, if the cuff is attached to the upper left arm, the second site may be the left hand or a finger of the left hand to which the second sensor is attached, or which is monitored by the second sensor.

The first and second signals are generally acquired and sampled synchronously. Other signals than ECG and PPG signals that are related to the subject's heart rate and allow the computation of PAT and/or PTT may be used in addition or as alternative, such as a sensor detecting heart sound, a sensor detecting chest vibrations, etc. The ECG and PPG signals may stem from different devices, e.g. the ECG may be acquired at the subject's chest and the PPG signal may be acquired at the subject's wrist. These signals should be synchronized. Synchronization involves two parts: aligning time point and aligning clock pacing. The ECG sensor may also be integrated in a watch, in which case both signals can be based on the same clock from the device and are synchronized within the electronics of the device in the usual manner.

Instead of two sensors, the two signals can also be derived by sensing one primary signal, representing the pulse's shape, and by extracting (at least) two sub-signals through pulse decomposition, or other method yielding a BP dependent surrogate. For example, by decomposition direct pulse (from heart to measurement location) and reflected pulse (from heart to reflection location to measurement location) can be extracted, and the time lag between them can serve as PAT/PTT.

The system 1 further comprises a device 50 disclosed herein and described in the following for calibrating a BP measurement function (e.g., a model or algorithm) from the reference BP value, a computed pulse-related value and a computed derivative value of a derivative of a fitting function at the reference BP value. Further, the device 50 may optionally be configured to determine and monitor BP of the subject by use of the BP surrogate. Furthermore, the device 50 may be configured to store the determined calibration and/or calibrated measurement function, and use them in further measurement of BP, based on the surrogate values. During such measurement, the cuff may be absent, but only the device 50 may be worn by the subject. Together with the sensors 30 and 40, the device 50 may be considered as measurement system.

The system 1 may further optionally comprise an output interface 60 configured to issue any determined information, such as BP values of the subject determined and monitored by use of the BP surrogate. The output interface 60 may generally be any means that outputs information in visual or audible form, e.g. in text form, as image or diagram, as sound or spoken words, etc. For instance, the output interface 60 may be a display, a loudspeaker, a touchscreen, a computer monitor, the screen of a smartphone or tablet, etc.

The system 1 may further optionally comprise a control unit 70 that is configured to control the pressure-delivery system 10, e.g. based on a control signal provided by the device 50 or computed by the system 1. The control unit 70 may be part of the device 50 or may be an external entity.

Fig. 7 shows a diagram of an embodiment of a device 50 for calibrating a BP measurement function for use in measuring / monitoring a subject's blood pressure according to the present invention.

The device 50 comprises a pressure input 51 configured to obtain time-dependent cuff pressure values during inflation and/or deflation of a cuff 11 of a pressure-delivery system 10 attached to a subject's body part. The device 50 further comprises a sensor input 52 configured to obtain a first time-dependent sensor signal and a second time-dependent sensor signal, both related to the subject's heartbeat and measured, preferably at different sites of the subject's body, during the inflation and/or deflation of the cuff. The BP input 51 and the sensor input 52 may be directly coupled or connected to the cuff 11 and the first and second sensors 30, 40 or may obtain (i.e. retrieve or receive) these signals from a storage, buffer, network, or bus, etc. The inputs 51 and 52 may thus e.g. be (wired or wireless) communication interfaces or data interfaces, such as a Bluetooth interface, Wi-Fi interface, LAN interface, HDMI interface, direct cable connection, or any other suitable interface allowing signal transfer to the device 50.

The device 50 further comprises a processing unit 53. The processing unit 53 may be any kind of means (remote or local) configured to process the signals and determine the calibration of the BP measurement function. Further, it may be configured to determine and monitor BP of the subject by use of the BP surrogate, for which purpose the subject is not required to wear the cuff. It may be implemented in software and/or hardware, e.g. as a programmed processor or computer or app on a user device such as a smartphone, smartwatch, tablet, laptop, PC, workstation, etc.

The device 50 may further comprise an output 54 configured to output any determined information. The output 54 may generally be any interface that provides the determined information, e.g. transmits it to another device or provides it for retrieval by another device (e.g. a smartphone, computer, tablet, etc.). It may thus generally be any (wired or wireless) communication or data interface.

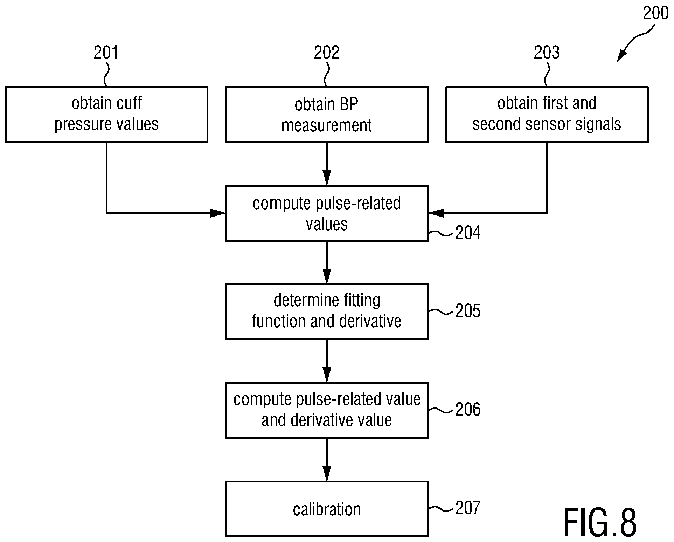

Fig. 8 shows a diagram of an embodiment of a method 200 according to the present invention. The steps of the method 200 may be carried out by the device 50, wherein the main steps of the method are carried out by the processing unit 53. The method may be implemented as computer program running on a computer or processor.

In a first step 201 time-dependent cuff pressure values during inflation and/or of a cuff of a pressure-delivery system attached to a subject's body part are obtained. In a second step 202 a reference BP value is obtained or computed. Typically, the reference BP value, such as DBP or MAP, is determined from the cuff pulsing, or determined by the cuff's system. Cuff pulsing (pulsing with every heartbeat) is a signal superimposed on the measured cuff pressure. DBP and MAP may be used to define the fitting range. As discussed above, the reference BP value is typically equal to DBP, but may also be determined from the (PAT, CP) data (i.e., not from the cuff pulsing).

In a third step 203 a first time-dependent sensor signal and a second time-dependent sensor signal, both related to the subject's heartbeat (i.e., the measured signals relate to the (heart) beating that induces a (propagating) pulsing in the vascular system) and preferably measured at different sites of the subject's body during the inflation and/or deflation of the cuff, are obtained. It shall be noted that the order of the steps 201 to 203 does not indicate a chronological order, but these steps may be carried out in any chronological order. Preferably, steps 201 and 203 are carried out simultaneously (their outputs may be synchronized, such that in a next step their values can be paired accordingly), and step 202 is carried out in advance or after steps 201 and 203.

In a fourth step 204, during inflation and/or deflation of the cuff, pulse-related values are computed from the first and second time-dependent sensor signals using a first feature in the first time-dependent sensor signal and a second feature in the second time-dependent sensor signal, the pulse-related values being PAT values or PTT values. PAT or PTT values may be extracted via appropriate feature extraction techniques from the first and second signals (e.g. ECG and PPG signals). Other features may also be derived from the single signals such as heart rate, PPG morphology features, etc. The pulse-related values computed in step 204 are preferably paired with time-dependent cuff pressure values obtained in step 201.

Next to using travel time (pulse velocity) as surrogate, another surrogate resides in the pulse shape. In the latter the ECG signal is not used. For example, the pulse shape may be decomposed in a set of (superimposed) pulses, where one can be identified as being due to reflections. The primary and secondary (decomposed) pulses reflect a travel time (the term "travel" being used to distinguish from the reserved term "transit" in PTT). Other characteristics are in amplitudes and their ratios of the (decomposed) pulses.

In a fifth step 205, a fitting function and its derivative between the pulse-related values and the cuff pressure values are determined from selected pairs of pulse-related values and corresponding temporally related cuff pressure values.

In a sixth step 206, the pulse-related value and a derivative value of the derivative of the fitting function at a reference BP value are computed.

In a seventh step 207 a BP measurement function, relating BP surrogate (of the same nature as the pulse-related values in step 204) to a BP estimate (the value to report to the user as BP measurement), is calibrated from the reference BP value, the computed pulse-related value and the computed derivative value of the derivative of the fitting function at the reference BP value.

Thus, according to a first main aspect of the present invention a BP measurement system that senses a BP surrogate where the prediction/mapping function from sensed value (the surrogate) to (hence, estimated) BP value is calibrated. BP can be tracked continuously thereafter. Criteria are used in an embodiment to include cuff pressure ranges during inflation and/or deflation to reliably calibrate BP surrogates from cuff-pressure / BP surrogate data pairs. Conventionally, calibration requires a complete cuff inflation. For PAT-based surrogate calibration from a continuous BP measurement, as used in an embodiment, only PAT values induced by cuff pressure levels are utilized given by well-defined criteria fulfilling physiological constraints. Thus, certain data are discarded according to an embodiment.

As mentioned above, methods using wearables, such as smart watches, are more and more used for determining a subject's BP. Typically, a PPG sensor is hosted in the device, which measures the reflection intensity of light emitted by LEDs (and captured by photodiodes). Typical wavelengths are green, red, and infra-red. The PPG signal responds to the pulsing of the blood passing by beneath the device, which pulsing is due to the beating of the heart. The traversal of pulses through the arteries as well as their shape depends on several factors, of which blood pressure (BP) is one. The dependence on other factors, such as age, length, elasticity of the artery's wall, wall thickness, and artery's diameter, imply that for best accuracy the measurement system needs to be calibrated to its particular user.

In particular, the elasticity of the artery's wall determines to great extent the pulse's propagation. The propagation velocity can be described by the Moens-Korteweg and the Bramwell-Hill equation, which model pulse propagation under some assumptions (such as constant diameter and viscosity-free propagation). The two are equivalent but use different quantities as their independent variables: elasticity and compliance, respectively. The two can be expressed in each other. Elasticity and compliance depend on blood pressure. Consequently, through this dependence, the propagation velocity depends on blood pressure. Since a pulse may be composed of several base pulses (e.g., the primary one and reflected ones from other branches in the arterial system), each yielding another transition to the point of observation, the superimposed pulse shape is dependent on blood pressure. The propagation mechanism itself may also shape the pulse.

The propagation velocity, or a change in it, is measured by observing the times at which a pulse (due to a same beat) passes by at two different locations. In a PTT based embodiment, typically two PPG sensors are used, at two body locations (e.g. finger and toe). In a PAT based embodiment, one PPG sensor (e.g., at wrist, ear, chest, finger, toe) in conjunction with an ECG sensor is used. Typically, the R-peak in the ECG signal is used as one time moment, where a fiducial point in the PPG signal provides the other. Typical fiducial points are the foot, the point of steepest ascent (largest derivative), and the peak in the PPG signal. Preferred are foot and steepest ascent.

When BP increases, the artery's elasticity index (Young's modulus) increases, or compliance decreases, and the arrival time PAT decreases (shorter transition time). By calibrating the relationship between BP and PAT, $BP = p(PAT)$, blood pressure can be determined by measuring the PAT.

One approach to obtain the calibrated curve is by measuring PAT for varying values of BP, and to determine the relationship by fitting the curve to the obtained datapoints. In fitting, a model is assumed, and this model's parameters are determined for minimizing some error criterion. The fitting poses the problem of obtaining datapoints over a range of BP values. Typically, for the model a polynomial relation is used, but more sophisticated models are feasible, in particular when the underlying physics and physiology suggest so. Commonly, such models are generally known in the art and validated.

From theoretical considerations, backed by experimental verification, expressions for the function $p(.)$ can be derived. For example, mentioned Moens-Korteweg and Bramwell-Hill equation can be part of this derivation, together with equations that relate the elasticity index to BP. Example equations include:

$$(1) \qquad BP = BP_0 + p'(PAT_0) . (PAT - PAT_0)$$

$$(2) \qquad BP = BP_0 + \left( \frac{L}{PAT - PEP} - \frac{L}{PAT_0 - PEP} \right) = BP_1 + \frac{L}{PAT - PEP}$$

$$(3) \qquad BP = BP_0 + m.\log\left(\frac{L}{PAT-PEP}\right) - m.\log\left(\frac{L}{PAT_0-PEP}\right) = BP_0 + m.\log\left(\frac{PAT_0-PEP}{PAT-PEP}\right)$$

In these equations, $BP$ and $PAT$ are the variables; the others, $BP_0$, $BP_1$, $p'(PAT_0) = \frac{dp(PAT_0)}{dPAT}$, $PAT_0$, $L$, $PEP$, m are parameters, i.e. constants to be determined. $BP$ equals $BP_0$ when $PAT$ equals $PAT_0$. $L$ represents the path length over which the pulse transits, $PEP$ stands for Pre-Ejection Period and reflects the time between R-peak in the ECG signal, where contraction starts, and the actual ejection of the blood pulse into the artery, where transition starts.

The values of the constants are from external source or are determined by the calibration procedure. Examples of external sources include literature, e.g., studies by others and handbooks. The values themselves might be parametrized, e.g., L may be expressed as a fraction of the user's height (which fraction's value can be found in the Biomechanics literature). Other of such parameters may be age, gender, region, etc. These parameter data may have a default value or are provided by the user. See also the description below following equation (4).

Either of these (or other) equations can serve as model in the measurement system, such that for a measured $PAT$ a corresponding blood pressure $BP$ can be returned. The parameters are determined from calibration. Measuring at a given moment would yield a single datapoint, i.e., a $BP_0$ with corresponding $PAT_0$. The other parameters stay undetermined.

It is known that when a cuff is applied to the upper arm and the cuff is inflated an increase in $PAT$ with increasing $CP$ will be observed. Typically, CP has been directly associated with BP. For example, in Y.S. Yan and Y.T. Zhang, A Novel Calibration Method for Noninvasive Blood Pressure Measurement Using Pulse Transit Time, Proc. 4th IEEE-EMBS Int. Summer School and Symp. Med. Devices and Biosensors, Cambridge, UK, Aug. 19-22, 2007, a blood pressure equal to $(BP - CP)$ is associated with the $PAT$ at that $CP$ value.

However, it has been found that it is inaccurate to associate CP directly with BP. When the cuff inflates the BP does not change, other than through secondary effects such as the occlusion of the veins. The occlusion will stop the flow, not immediately the pulsing, and by that induce some raise in the mean arterial pressure in the arm's (brachial, radial, ulnar) arteries.

Therefore, according to an embodiment of the present invention, it is proposed instead, to use the trend in the way the increasing $CP$ does change the $PAT$. This is based on the observation that at a point $BP_{ref}$ (herein also referred to as "reference BP value") the transmural pressure $P_{tm}$ changes in similar fashion, whether by increase in $BP$ or by decrease in $CP$. The transmural pressure is defined as the difference between the pressure inside and the pressure outside the artery: $P_{tm} = BP - CP$. This shows that a small change in $BP$ or $CP$, at the same reference $BP_{ref}$, will have the same effect on changing the transmural pressure, with opposite signs. The elasticity index or the compliance, at a given reference $BP_{ref}$, depends on the $P_{tm}$. As a consequence, the change in $PAT$ (at that $BP_{ref}$) will be the same in either case ($CP$ changes or $BP$ changes). It is noted that, typically, elasticity index or the compliance are presented in relation to the blood pressure, but in those cases, there is no cuff present, $CP = 0$, and $P_{tm} = BP$.

The relationship c(.) how $PAT$ changes with cuff pressure $CP$ is denoted as $PAT = c(CP)$, and the relationship $p(.)$ how a measured $PAT$ predicts actual $BP$ is denoted as $BP = p(PAT)$. Then, the above argument on equality of the change in $P_{tm}$ by a small change in either $CP$ or $BP$ at reference $BP_{ref}$ implies that the derivatives of the two relationships can be equated at that reference $BP_{ref}$ as:

$$(4) \qquad p'\left(c\left(BP_{ref}\right)\right) = -\left\{c'\left(BP_{ref}\right)\right\}^{-1}.$$

This means that an intercept of the BP prediction model is determined by a pulse-related value at the reference BP value and a slope of the BP prediction model at said intercept is determined from the slope of the fitting curve at the reference BP value, in particular by the reverse inverted (reciprocal) and negated slope of the fitting curve at the reference BP value.

Since $c(.)$ relates $PAT$ to pressure and $p(.)$ relates pressure to $PAT$, the reciprocal derivative is used. A refinement of Eq. (4) is to account for the length of path over which $p(.)$ and $c(.)$ are affected: typically the length $L$ from heart to device for $p(.)$ and the length of the cuff $L_{cuff}$ for $c(.)$, This is reflected by Eq. (6) below.

Preferably, the reference $BP_{ref}$ is chosen at a cuff pressure at which deformation or other distortions of the artery beneath the cuff are minimal, such that effects by those deformations are not yet present in the measured $PAT$. This is in line with the principle to observe the differential change (the derivative) rather than a change averaged over an interval. Therefore, $BP_{ref} = DBP$ is a preferred choice. At that reference $BP_{ref}$, it holds that $p(c(BP_{ref})) = BP_{ref}$.

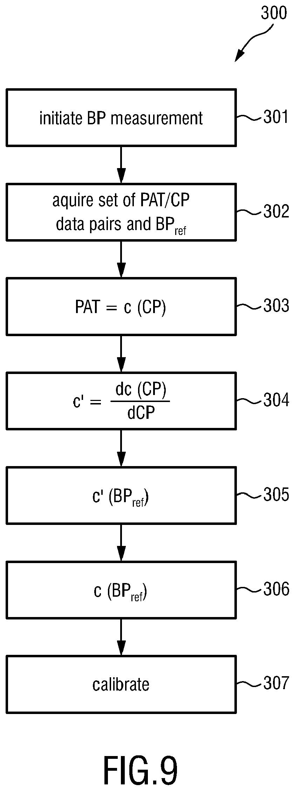

Then, taken together, the following calibration procedure 300 is proposed in an embodiment as depicted in the flowchart shown in Fig. 9. In step 302, after initiating the BP measurement in step 301, via the cuff inflation data pairs ($PAT$, $CP$) are extracted. Furthermore, a reference BP value (or point) $BP_{ref}$ in that curve is obtained. Preferably, this is the point where the $PAT$ values start to change, which point is typically the $DBP$. Given these data pairs, in step 303 fitting is applied to find the relation $PAT = c(CP)$. Preferably, the data between $DBP$ and $MAP$ are used (polynomial fit), or all below $MAP$ (exponential or logarithmic fit). Alternatively, the range above (about) 30 mmHg (up to MAP) is used, to discard PAT changes due to the occlusion of the veins (which take the flow component out of the pulse propagation).

With the so-obtained function $c(.)$, the derivative $c'(CP) = \dfrac{dc(CP)}{dCP}$ is determined (step 304), and its value at $BP_{ref}$, i.e., $c'(BP_{ref})$ is determined (step 305). In step 306, the $PAT$ at $BP_{ref}$ is determined by substitution, i.e., $c(BP_{ref})$.

Subsequently, in step 307 the determined $BP_{ref}$, $c(BP_{ref})$, and $c'(BP_{ref})$ are used to calibrate a chosen BP prediction model $BP = p(PAT)$: The intercept of $p(.)$ is given by the $PAT$ at $BP_{ref}$, i.e., $p(c(BP_{ref})) = BP_{ref}$, and the slope of $p(.)$ at that intercept follows as: $p'\left(c\left(BP_{ref}\right)\right) = -\left\{c'\left(BP_{ref}\right)\right\}^{-1}$.

Next to determining $DBP$ from the measured CP values, e.g., by applying the oscillometric method to the pulsing in the cuff pressure signal, $DBP$ can also be taken from the cuff's internal measurement system.

The model p(.), e.g., Eqs. (1)-(3) used in step 307, is not to be confused with the model $c(.)$, e.g. polynomial or exponential, used in step 303 in the fitting of the cuff inflation data.

Thus, instead of calibrating at a single point, since there is no range of varying BP values, two measurement points are available, and, combined with the used model $p(.)$, the accuracy of the calibration is improved. For example, the linear model Eq. (1) above is fully determined by its parameters $BP_0$, $PAT_0$, and $p'(PAT_0)$. For the model Eq. (2), preferably either $L$ or $PEP$ are assigned an assumed value, e.g., taken from measurements over a comparable population, the other determined by equating the derivatives as explained. For example, $L$ may be adapted based on a humanoid model and asking the user to provide his/her length. For the model Eq. (3) either the parameter $m$ or $PEP$ needs to be assigned an assumed number. For example, $PEP$ can be taken a typical (average, moderate) number from literature, perhaps dependent on user age. Instead of assuming the unknown parameters, another method is to equate higher-order derivatives of $c(.)$ and $p(.)$.

In the case of model Eq. (2), $L$ can be estimated using the model and knowing the length of the cuff $L_{cuff}$. By differentiation of the model, $BP = BP_1 + \dfrac{L}{PAT}$, it follows that

$$(5) \qquad \frac{dBP}{dPAT} = -\frac{L}{PAT^2}.$$

The calibration hypothesis Eq. (4) states $\frac{dBP}{dPAT} = -\left\{\frac{dPAT}{dCP}\right\}^{-1}$, where $\frac{dPAT}{dCP} = c'(.)$ is obtained from the measurements during the cuff inflation. Since, in first order, the cuff only alters the propagation along the path below the cuff, and since the transition time *PAT* is proportional with path length, a refinement of Eq. (4) would be:

$$(6) \qquad \frac{dBP}{dPAT} = -\left\{\frac{dPAT}{dCP} . \frac{L}{L_{cuff}}\right\}^{-1}, \text{ at } BP_{ref}, PAT_{ref} = c(BP_{ref})$$

Substituting Eq. (6) into Eq. (5) yields, after some mathematical rewrite,

$$(7) \qquad L = PAT_{ref}\sqrt{\frac{L_{cuff}}{c'(BP_{ref})}}.$$

An alternative approach to estimate the length *L* includes observing the pulsing in the cuff pressure signal, next to the ECG and PPG signals. The path that the pulse is traversing can be written as $L_{ep} = L_{ec} + L_{cp}$, where the subscript e refers to the R-peak in the ECG signal, i.e., located at the heart, the subscript c refers to the cuff location, and the subscript p to the wrist (PPG signal). Assuming constant propagation velocity, it follows that $PAT_{ep} = PAT_{ec} + PAT_{cp}$. This assumption is reasonable at the onset of the cuff inflation, i.e. at $BP_{ref}$. If the length of cuff to wrist, $L_{cp}$, is measured, the length *L* follows as

$$(8) \qquad L = \frac{PAT_{ep}}{PAT_{cp}} . L_{cp}$$

In order to obtain an accurate fit for the dependency of *PAT* on *CP* during cuff inflation, i.e., for the function *c*(.), preferably the fitting range is restricted for *CP* values between *DBP* and *MAP*, *DBP* < *CP* < *MAP*. Experimental results have indicated that above *MAP* there is more scattering in the data points, leading to more noise in the fitting process. At *DBP* the curve tends to have a strong change. When limiting above *DBP*, a polynomial fitting can be applied. Alternatively, when including the data points below *DBP*, an exponential (logarithmic) model could be used in the fitting. In an embodiment it is preferred to use a fitting method in which the error criterion observes the error in both the *CP* and in the *PAT* values. Such methods are known as TLS (Total Least Squares) and ODR (Orthogonal Distance Regression). Where the range is determined by the error in the fitting procedure, known criteria may be used, such as goodness-of-fit and standard error. The range may be selected for minimizing that error (while maximizing the range).

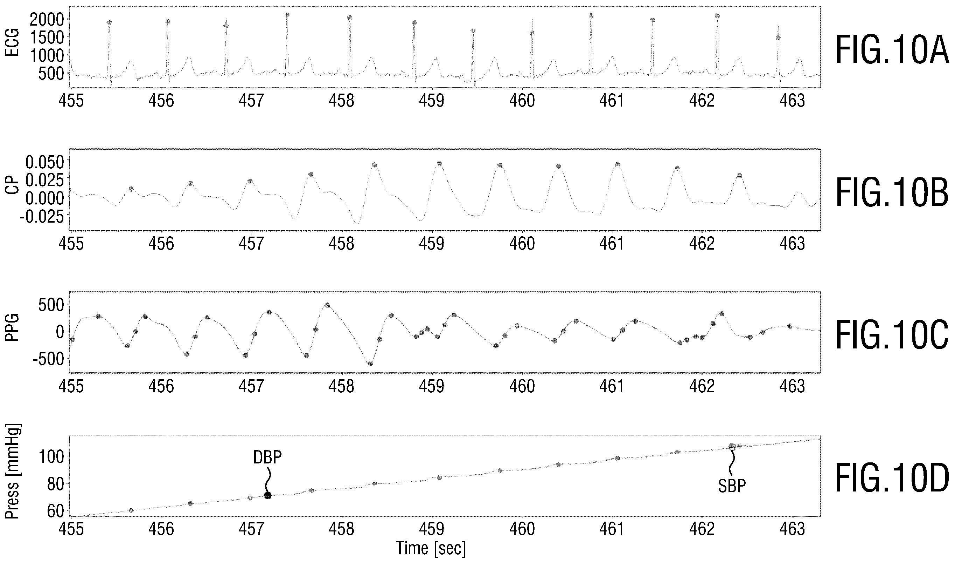

Fig. 10 shows diagrams of different signals over time used according to an embodiment of the present invention. Fig. 10A shows an ECG signal with marker at the R-peaks. Fig. 10B shows the pulsing in the cuff pressure signal (CP) with marker at every peak (e.g., as a bandpass filtered version of the cuff pressure shown in Fig. 10D). Fig. 10C shows a PPG signal with marker at foot, steepest ascent, and peak. Fig. 10D shows cuff pressure (inflating cuff) with marker when cuff pressure reached DBP and SBP. PAT values are determined as the time difference between R-peak and one of fiducial points in the PPG signal.

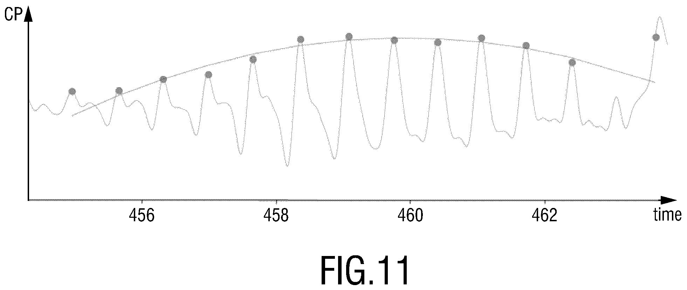

Fig. 11 shows a diagram of the pulsing of the cuff pressure. It exemplifies how DBP, MAP, and SBP can be determined, as known in the art. Pulsing reaches a maximum when cuff pressure is at about MAP. DBP is found as the cuff pressure when the pulsing is, for example, at 0.717 of this maximum (left side; cuff pressure below MAP). SBP is found as the cuff pressure when the pulsing is, for example, at 0.593 of this maximum (right side; cuff pressure beyond MAP).

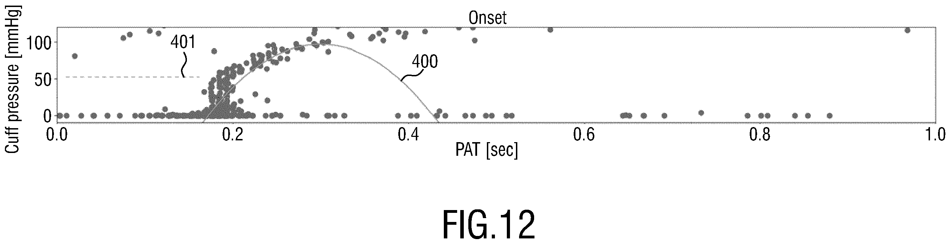

Fig. 12 shows a typical plot of PAT against CP. When CP increases, the PAT also increases. Curve 400 is a polynomial (quadratic) fit of c(.). The dashed line 401 is at about $BP_{ref}$ (DBP for this user). The polynomial fit is more accurate if values above DBP are used. Alternatively, a logarithmic or exponential fir may be applied over the full range (both up to MAP).

The described calibration method establishes the parameters of an assumed prediction model, predicting *BP* from a measured *PAT*. It also makes use of a reference point $BP_{ref}$, which is typically the *DBP*. Therefore, the model best predicts DBP. Additional measures can be taken to also predict the SBP. For example, by observing the size in foot-to-peak in the PPG signal, *ftpSz,* the SBP can be computed as the DBP plus an additional term proportional to that foot-to-peak size:

$$(9) \qquad SBP = DBP + \alpha . ftpSz$$

where *a* is also determined during calibration (determine average foot-to-peak sizes together with the DBP and SBP of that user). Instead of using the *ftpSz* at every beat per computation, some smoothing is preferred. For example, the median over the *ftpSz* in a 11-point window centered around the current beat could be used.

In another embodiment, a control signal for controlling the pressure-delivery system may be computed to repeatedly inflate its cuff in partial inflations up to a cuff pressure below the subject's systolic BP. The pump control may be configured to repeatedly apply a short increasing pressure profile (pressure ramps) ranging from 0 mmHg cuff pressure to approximately diastolic pressure level or until a pulse amplitude change in the PPG signal is detected, thereby recording cuff pressure and PAT, preferably with constant pressure between the ramps, such that more stable and multiple PAT - CP pairs are obtained. The processing may conduct parameter regression per applied pressure ramp and average the obtained regression parameters to improve the overall calibration accuracy.

This embodiment is based on the idea that only low cuff pressure levels should be used for calibration. Repeated sampling of those relevant cuff pressure levels is feasible in a short enough time period, such that the BP level is stable and does not change, which would distort the calibration process. Furthermore, repeated inflation ramps even without a pause in between are enabled by low maximum pressure. There is no need to wait for tissue and arteries to recover from high pressurization (i.e. there is no hysteresis effects). Another advantage is that cuff pressure levels below diastolic pressure are very unobtrusive for the patient, particularly for awake patients, resulting in less discomfort and less stress to skin than full inflation ramps up to supra-systolic levels. Still another advantage of such an embodiment is that it does not pose extra requirements on the pressure pump, which is integrated into a NIBP device. An alternative proposal to achieve the same effect by means of a single slow pressure ramp from 0 mmHg to diastolic pressure level would require a pump that can achieve very small air flow at low pressure levels, which would represent a significant challenge to the pump design and is currently not supported by standard pumps used in NIBP devices.

This idea does not apply only to systems using PAT measured by ECG and PPG sensors, but more generally to systems with alternative methods for measuring pulse propagation delay (e.g. based on differential pulse transit time) and with a pressurizable cuff being applied within the propagation path of considered pulse waves.

In the following embodiments to achieve improved BP surrogate calibration via repeated partial inflations are explained. In these embodiments PAT values are inferred on a beat-by-beat basis using the PPG sensor and the ECG sensor.

In an embodiment a baseline value $PAT_{base}$ for PAT is obtained by averaging the PAT measurements over a certain period. That period can be predetermined and fixed or set by user, e.g. to 20s or 15s or any other sufficiently long period in time to sufficiently suppress unwanted fluctuations caused by e.g. respiration (typical frequency of 0.1-0.5 Hz), Meyer waves (typical frequency of 0.1 Hz), or other measurement noise.

By activating the pump of the NIBP device a first (partial) inflation ramps up to a certain sub-systolic level is conducted. This can be diastolic pressure (known from the last NIBP measurement), some specified sub-diastolic level, a certain fixed cuff pressure level (e.g. 60 mmHg or 40 mmHg), or a pressure level where PAT has exceeded a certain threshold compared to $PAT_{base}$ or a pre-defined amplitude change of the PPG amplitude is detected. The inflation speed may be set to a fixed inflation speed, such as e.g. 10 mmHg/s, or otherwise chosen, e.g. dependent on the heart rate to guarantee a certain minimum number of heart beats within the inflation ramp. During cuff inflation, cuff pressure $p_{cuff}$ is measured at the same time instances where PAT values are measured.

The measured data pairs (PAT, C) may be used for conducting a parameter regression (curve fitting) of the unknown model parameters $m_1, m_2, ..., m_N$ of the parameterized model function f, e.g., Nth-order polynomial, that models the relation between PAT and cuff pressure CP around an operating point $BP_{ref}$. Therefore, the regression equation is obtained as follows:

$$PAT = L_{cuff} \cdot f(CP \quad ; m_1, m_2, ..., m_N).$$

As an example, in a simple linear proportional model the number of unknown model parameters would be two (f = $m_1 \cdot CP + m_2$). A criterion for parameter regression is the least square error, however, parameter regression could be conducted according to different error measures, such as e.g. least absolute error or total least square (TLS) fitting.

In order to improve parameter regression, the cuff is deflated, preferably a rest is incorporated before another partial inflation is conducted, and another parameter regression is performed, or the data are combined in a single regression. This can be repeated multiple times. In preferred embodiment at least one extra partial inflation is conducted. In case the data are not combined in a single regression, the obtained regression values for the model parameters are averaged, thereby improving the accuracy of model-parameter calibration. The degree of variation of obtained regression values can be used to decide how many repeated partial inflations shall be conducted. For example, if the obtained regression values after two partial inflations closely align, no further partial inflation is needed. It may be useful to inflate the cuff only up to MAP. This improves convenience to the user, especially when the cuff is inflated multiple times. It will also induce less adaptations by the vascular system due to the cuff pressure (e.g., vasodilation) and speed up measurements.

In order to calibrate the resulting continuous BP estimator a NIBP measurement is conducted, yielding a blood pressure reference measurement BP$_{ref}$. This is enabled by continuing the last partial inflation to supra-systolic cuff pressure level, such that a standard inflation-based oscillometric blood pressure measurement can be computed. In other words, the last inflation ramp is a 'full' cuff inflation ramp.

Typically, calibration may be repeated at regular intervals, i.e., the steps required for calibration are repeated from time to time to recalibrate the continuous blood pressure estimator. This can be done according to some regular schedule, or in case significant blood pressure changes occur. In general, the proposed pressure profile comprising repeated partial inflations can be performed whenever an NIBP measurement is triggered automatically or manually.

In an alternative embodiment the partial inflations (optionally with some pause (e.g. 5-30 sec) between the inflations to allow the vascular system to (re)stabilize from the cuff pressure) are not prepended but appended. This profile has the advantage that the NIBP value can be displayed sooner compared to the previous embodiment of the pressure profile with appended partial inflations. However, the time distance between the first parameter calibration and the second parameter calibration is longer. If the patient's hemodynamic status is not constant during this time, i.e., if the model parameters are changing, the gain in accuracy by averaging over multiple calibration events is diminished.

Another benefit of partial inflations, next to less disturbing the vascular system and annoying the user, is that the measurement itself lasts shorter. As with stepwise increments of cuff pressure, an instrumented cuff may be used since conventional cuffs typically have a fixed inflation speed, which may possibly still need modification to provide the cuff pressure values and include synchronization means.

In another alternative embodiment improved parameter regression is not obtained by averaging over the regression parameters from multiple partial inflations, but rather by using all obtained data pairs (*PAT*, *CP*) from multiple partial inflations for a single regression. For this option, the accuracy gain is obtained by having more regression data points available for a single regression. Furthermore, instead of predetermining the number of conducted partial inflations, it could rather be determined though threshold comparison of the regression error. If the regression error drops below that threshold, no further partial inflation is needed.

Still another alternative embodiment further provides that after the continuous blood pressure estimator has been calibrated via model-parameter regression and NIBP reference measurement, only partial inflations are conducted, e.g. every 10 minutes. In ambulatory use, re-calibrations will be made less often since the user will be 'cuff-free' most of the time and BP is much more stable. This may be done as explained above. A 'full calibration' with NIBP reference measurement is only conducted if the model-parameter regression results in a relevant change of model parameters $m_1, ... m_N$, compared to the parameter regression result associated with the last 'full calibration'. If no relevant change is detected, the parameters of the current continuous blood pressure estimator are not updated and no full inflation for NIBP measurement is conducted.

The present invention applies to the broad range of applications, e.g. for in-hospital monitoring settings, where NIBP, ECG, and PPG sensors are already available and used. For example, these sensors are virtually used in all settings in the OR and the ICU, but this includes also certain settings in the general ward as well as settings in specialized wards like neurology. More specifically, it allows for measuring beat-to-beat arterial blood pressure non-invasively. Beat-to-beat blood pressure monitoring can provide early detection of hypotensive events, which are associated with adverse patient outcome. Furthermore, it can provide an early warning for hemodynamic instability. The disclosed apparatus and method may also be applied for long-term monitoring, e.g. for 24h BP monitoring or BP monitoring at night during sleep.

The present invention may preferably be used in (medical) wearables for vital signs, particularly for NIBP/PAT. Common form factors include chest-based patches, such as Philips AMD MCOT, and smart bracelets/watches, such as Philips Health Band or other (wrist-worn) devices. Other wearable form factors may be considered as well, including, e.g., hearables, distributed patches, smart glasses (with built-in continuous contact-based or contactless PPG and spot ECG), etc.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1.  A device (50) for calibrating a blood pressure, BP, measurement function, the device comprising:

    a pressure input (51) configured to obtain time-dependent cuff pressure values during inflation and/or deflation of a cuff (11) of a pressure-delivery system (10) attached to a subject's body part;
    a sensor input (52) configured to obtain a first time-dependent sensor signal and a second time-dependent sensor signal, both related to the subject's heartbeat and measured during the inflation and/or deflation of the cuff; and
    a processing unit (53) configured to:

    - compute pulse-related values from the first and second time-dependent sensor signals measured during inflation and/or deflation of the cuff, using a first feature in the first time-dependent sensor signal and a second feature in the second time-dependent sensor signal, the pulse-related values being pulse arrival time, PAT, values or pulse transit time, PTT, values,
    - determine a fitting function and its derivative between the pulse-related values and the cuff pressure values from selected pairs of pulse-related values and corresponding temporally related cuff pressure values,
    - compute the pulse-related value and a derivative value of the derivative of the fitting function at a reference BP value, and
    - calibrate the blood pressure measurement function based on the reference BP value, the computed pulse-related value and the computed derivative value of the derivative of the fitting function at the reference BP value.

2.  The device according to claim 1,
    wherein the processing unit (53) is configured to obtain or determine the diastolic BP, DBP, as reference BP value, in particular to determine DBP from selected pairs of pulse-related values and corresponding temporally related cuff pressure values or to obtain DBP from an internal measurement of the cuff.

3.  The device according to claim 1 or 2,
    wherein the processing unit (53) is configured to calibrate a chosen BP prediction model having an intercept and a slope, wherein an intercept of the BP prediction model is determined by a pulse-related value at the reference BP value and a slope of the BP prediction model at said intercept is determined from the slope of the fitting curve at the reference BP value, in particular by the reverse inverted, reciprocal and negated slope of the fitting curve at the reference BP value.

4.  The device as claimed in any one of the preceding claims,
    wherein the processing unit (53) is configured to use only pairs of pulse-related values and corresponding cuff pressure values for computing a calibration parameter for the BP measurement function that have been computed from the first and second time-dependent sensor signals measured during a partial inflation of the cuff.

5.  The device as claimed in in any one of the preceding claims,
    wherein the processing unit (53) is configured to use only pairs of pulse-related values and corresponding cuff pressure values for computing a calibration parameter for the BP measurement function for which a peripheral BP is substantially constant, in particular varies by less than 10 % or less than 5 %.

6.  The device as claimed in any one of the preceding claims,
    wherein the processing unit (53) is configured to use only pairs of pulse-related values and corresponding cuff pressure values for computing a calibration parameter for the BP measurement function for which the cuff pressure value is below the subject's diastolic BP, in particular the subject's latest measured diastolic BP, or below the subject's mean BP, in particular the subject's latest measured mean BP, or below a set threshold cuff pressure.

7.  The device as claimed in any one of the preceding claims,
    wherein the processing unit (53) is configured to use only pairs of pulse-related values and corresponding cuff pressure values for computing a calibration parameter for the BP measurement function for which the cuff pressure value is in a range between a set minimum cuff pressure and a set maximum cuff pressure, in particular wherein the minimum cuff pressure is set in the range of 0 to 40 mmHg and the maximum cuff pressure is set in the range of 40 to 110 mmHg.

8.  The device as claimed in any one of the preceding claims,

wherein the processing unit (53) is configured to use only pairs of pulse-related values and corresponding cuff pressure values for computing a calibration parameter for the BP measurement function for which the pulse-related values are substantially constant, in particular vary by less than 10 % and/or to use only pairs of pulse-related values and corresponding cuff pressure values for computing the calibration parameter for the BP measurement function until the slope of a curve of the pulse-related values over time exceeds a threshold.

9. The device as claimed in any one of the preceding claims,
wherein the processing unit (53) is configured to compute a control signal for controlling the pressure-delivery system (10) to inflate its cuff (11), in particular to fully inflate the cuff for acquisition of the BP measurement value and to only partly inflate the cuff for acquisition of the first time-dependent sensor signal and the second time-dependent sensor signal.

10. The device as claimed in any one of the preceding claims,
wherein the first time-dependent sensor signal is an ECG signal and/or the second time-dependent sensor signal is a photoplethysmography, PPG, signal, in particular a contact PPG signal or a remote PPG signal.

11. The device as claimed in claim 1,
wherein the processing unit (53) is configured to use as reference BP value one of a value of zero, venous occlusion pressure, diastolic blood pressure, systolic blood pressure or mean arterial pressure.

12. The device as claimed in any one of the preceding claims,
wherein the processing unit (53) is configured to compute a higher-order derivative of the fitting and prediction functions and use them to calibrate the prediction function.

13. A system for calibrating a blood pressure, BP, measurement function, the system comprising:

a pressure-delivery system (10) comprising a cuff (11) configured to be attached to the subject's body part and to deliver a pressure to the subject's body part by inflating the cuff;
a pressure sensor (20) configured to acquire time-dependent cuff pressure values during inflation and/or deflation of a cuff of the pressure-delivery system attached to the subject's body part and to acquire or infer a reference BP value;
a first sensor (30) configured be attached to a first site of the subject's body and to acquire a first time-dependent sensor signal related to the subject's heartbeat during the inflation and/or deflation of the cuff;
a second sensor (40) configured be attached to a second site of the subject's body and to acquire a second time-dependent sensor signal related to the subject's heartbeat during the inflation and/or deflation of the cuff; and
a device (50) as claimed in any one of the preceding claims for calibrating a blood pressure measurement function from the reference BP value, the time-dependent cuff pressure values and the first and second time-dependent sensor signals.

14. A method of calibrating a blood pressure, BP, measurement function, the method comprising:

- obtaining time-dependent cuff pressure values during inflation and/or deflation of a cuff (11) of a pressure-delivery system (10) attached to a subject's body part;
- obtaining a reference BP value;
- obtaining a first time-dependent sensor signal and a second time-dependent sensor signal, both related to the subject's heartbeat and measured during the inflation and/or deflation of the cuff;
- computing pulse-related values from the first and second time-dependent sensor signals measured during inflation and/or deflation of the cuff, using a first feature in the first time-dependent sensor signal and a second feature in the second time-dependent sensor signal, the pulse-related values being pulse arrival time, PAT, values or pulse transit time, PTT, values;
- determining a fitting function and its derivative between the pulse-related values and the cuff pressure values from selected pairs of pulse-related values and corresponding temporally related cuff pressure values;
- computing the pulse-related value and a derivative value of the derivative of the fitting function at a reference BP value; and
- calibrating the blood pressure measurement function based on the reference BP value, the computed pulse-related value and the computed derivative value of the derivative of the fitting function at the reference BP value.

15. A computer program comprising program code means for causing a computer or processor to carry out the steps of the method as claimed in claim 14 when said computer program is carried out on the computer.

FIG.1

FIG.2

FIG.3A

FIG.3B

FIG.4

EP 4 613 185 A1

FIG.5A

FIG.5B

FIG.5C

FIG.6

FIG.7

FIG.8

300

initiate BP measurement — 301

aquire set of PAT/CP
data pairs and $BP_{ref}$ — 302

$PAT = c\,(CP)$ — 303

$c' = \dfrac{dc\,(CP)}{dCP}$ — 304

$c'\,(BP_{ref})$ — 305

$c\,(BP_{ref})$ — 306

calibrate — 307

FIG.9

FIG.10A

FIG.10B

FIG.10C

FIG.10D

Time [sec]

FIG.11

FIG.12

EP 4 613 185 A1

Europäisches
Patentamt

European
Patent Office

Office européen
des brevets

# EUROPEAN SEARCH REPORT

**Application Number**

EP 24 16 1670

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2023/072842 A1 (KONINKLIJKE PHILIPS NV [NL]) 4 May 2023 (2023-05-04) * page 2, line 20 – line 32 * * page 18, line 13 – line 17 * * figures 1-21 * | 1-15 | INV.<br>A61B5/022<br>A61B5/024<br>A61B5/00<br>A61B5/021 |
| A | US 2010/160798 A1 (BANET MATT [US] ET AL) 24 June 2010 (2010-06-24) * abstract * * figures 1-21 * * paragraph [0062] * * paragraph [0065] – paragraph [0074] * | 1-15 | |
| A | RAMAKRISHNA MUKKAMALA ET AL: "Toward Ubiquitous Blood Pressure Monitoring via Pulse Transit Time: Theory and Practice", IEEE TRANSACTIONS ON BIOMEDICAL ENGINEERING, vol. 62, no. 8, 1 August 2015 (2015-08-01), pages 1879-1901, XP055365254, USA ISSN: 0018-9294, DOI: 10.1109/TBME.2015.2441951 * abstract * * page 19 – page 21 * * figure 8 * | 1-15 | TECHNICAL FIELDS SEARCHED (IPC)<br><br>A61B |

The present search report has been drawn up for all claims

| Place of search | Date of completion of the search | Examiner |
|---|---|---|
| The Hague | 18 April 2024 | Lai, Marco |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

**EP 24 16 1670**

This annex lists the patent family members relating to the patent documents cited in the above-mentioned European search report.
The members are as contained in the European Patent Office EDP file on
The European Patent Office is in no way liable for these particulars which are merely given for the purpose of information.

**18-04-2024**

| Patent document cited in search report | Publication date | Patent family member(s) | Publication date |
|---|---|---|---|
| WO 2023072842 A1 | 04-05-2023 | EP 4173556 A1<br>WO 2023072842 A1 | 03-05-2023<br>04-05-2023 |
| US 2010160798 A1 | 24-06-2010 | CN 102811659 A<br>CN 105342591 A<br>EP 2519144 A1<br>HK 1218613 A1<br>SG 181970 A1<br>SG 10201501410U A<br>US 2010160798 A1<br>WO 2011082341 A1 | 05-12-2012<br>24-02-2016<br>07-11-2012<br>03-03-2017<br>30-08-2012<br>28-05-2015<br>24-06-2010<br>07-07-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2023072842 A1

**Non-patent literature cited in the description**

- **SHAO, J. et al.** *An Optimization Study of Estimating Blood Pressure Models Based on Pulse Arrival Time for Continuous Monitoring*
- **VERKRUYSSE et al.** Remote plethysmographic imaging using ambient light. *Optics Express*, 22 December 2008, vol. 16 (26), 21434-21445
- **Y.S. YAN** ; **Y.T. ZHANG**. A Novel Calibration Method for Noninvasive Blood Pressure Measurement Using Pulse Transit Time. *Proc. 4th IEEE-EMBS Int. Summer School and Symp. Med. Devices and Biosensors*, 19 August 2007